(19) Europäisches Patentamt
European Patent Office
Office européen des brevets

(11) Veröffentlichungsnummer: **0 206 999 B1**

(12) # EUROPÄISCHE PATENTSCHRIFT

(45) Veröffentlichungstag der Patentschrift: **14.08.91**

(51) Int. Cl.[5]: **C07D 405/04**, A01N 43/36

(21) Anmeldenummer: **86810273.2**

(22) Anmeldetag: **16.06.86**

Die Akte enthält technische Angaben, die nach dem Eingang der Anmeldung eingereicht wurden und die nicht in dieser Patentschrift enthalten sind.

(54) **Mikrobizide Mittel.**

(30) Priorität: **21.06.85 CH 2649/85**

(43) Veröffentlichungstag der Anmeldung:
**30.12.86 Patentblatt 86/52**

(45) Bekanntmachung des Hinweises auf die Patenterteilung:
**14.08.91 Patentblatt 91/33**

(84) Benannte Vertragsstaaten:
**AT BE CH DE FR GB IT LI LU NL SE**

(56) Entgegenhaltungen:
**EP-A- 0 130 149**
**EP-A- 0 133 247**
**DE-A- 2 927 480**

(73) Patentinhaber: **CIBA-GEIGY AG**
**Klybeckstrasse 141**
**CH-4002 Basel(CH)**

(72) Erfinder: **Nyfeler, Robert, Dr.**
**Bärenfelserstrasse 8**
**CH-4057 Basel(CH)**
Erfinder: **Ehrenfreund, Josef, Dr.**
**Amselstrasse 11**
**CH-4123 Allschwil(CH)**

**EP 0 206 999 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue substituierte 3-Phenyl-4-cyanopyrrol-Derivate, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen haben die allgemeine Formel I

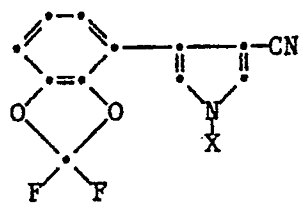

(I)

worin X Wasserstoff oder CO-$R_1$ ist, wobei $R_1$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, $C_1$-$C_6$-Alkoxy; $C_3$-$C_6$-Alkenyloxy, $C_3$-$C_6$-Cycloalkyl oder Tetrahydrofur-2-yl darstellt.

Unter dem Begriff Alkyl selbst oder als Bestandteil eines anderen Substituenten sind je nach der Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw.. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw. Unter Halogen soll hier und im folgenden Fluor, Chlor, Brom oder Jod, vorzugsweise Fluor, Chlor oder Brom, verstanden werden. $C_3$-$C_6$-Alkenyl steht für einen ungesättigten, aliphatischen Rest mit einer oder mit mehreren Doppelbindungen, so z.B. für Propenyl-(1), Allyl, Butenyl-(1), Butenyl-(2), Butenyl-(3), $CH_3CH=CHCH=CH$- usw. Unter Alkinyl sind ungesättigte, aliphatische Reste und maximal 6 Kohlenstoffatomen zu verstehen, z.B. Propargyl, Butinyl-(2), Butinyl-(3) usw.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Aufgrund ihrer ausgeprägten mikrobiziden Wirkung sind diejenigen Verbindungen der Formel I bevorzugt, die für X folgende Substituententypen oder Kombinationen dieser Substituententypen untereinander aufweisen:

Wasserstoff oder $COR_1$, wobei $R_1$ unsubstituiertes $C_1$-$C_4$-Alkyl oder durch Chlor, Brom oder $C_1$–$C_3$-Alkoxy substituiertes $C_1$-$C_4$-Alkyl; $C_3$-$C_4$-Alkenyl, $C_3$-$C_4$-Alkinyl, $C_1$-$C_4$-Alkoxy; $C_3$-$C_4$-Alkenyloxy, $C_3$-$C_6$-Cycloalkyl oder Tetrahydrofur-2-yl darstellt.

Unter den Verbindungen der Formel I sind insbesondere wegen ihrer hervorragenden fungiziden Eigenschaften folgende Einzelsubstanzen bevorzugt:

3-(2,2-Difluor-benzodioxol-4-yl)-4-cyanopyrrol (Verb. 1.1)
1-Acetyl-3-(2,2-difluor-benzodioxol-4-yl)-4-cyanopyrrol (Verb. 1.2)
1-Methoxyacetyl-3-(2,2-difluor-benzodioxol-4-yl)-4-cyanopyrrol (Verb. 1.15)
1-Methoxycarbonyl-3-(2,2-difluor-benzodioxol-4-yl)-4-cyanopyrrol (Verb. 1.24)
1-Allyloxycarbonyl-3-(2,2-difluor-benzodioxol-4-yl)-4-cyanopyrrol (Verb. 1.30)
1-n-Propoxyacetyl-3-(2,2-difluor-benzodioxol-4-yl)-4-cyanopyrrol (Verb. 1.32)

Die an erster Stelle genannte Verbindung besitzt darüber hinaus eine besondere Bedeutung als Zwischenprodukt zur Synthese weiterer fungizid-wirksamer Substanzen.

Die Verbindungen der Formel I werden erfindungsgemäss hergestellt:

a) in alkalischem Medium durch cyclisierende Michael-Addition des 2,3-(Difluormethylendioxy)-zimtsäurenitrils der Formel II mit p-Toluolsulfonylmethylisocyanid unter Abspaltung von p-Toluolsulfinsäure bzw. ihres Salzes in einem organischen Lösungsmittel:

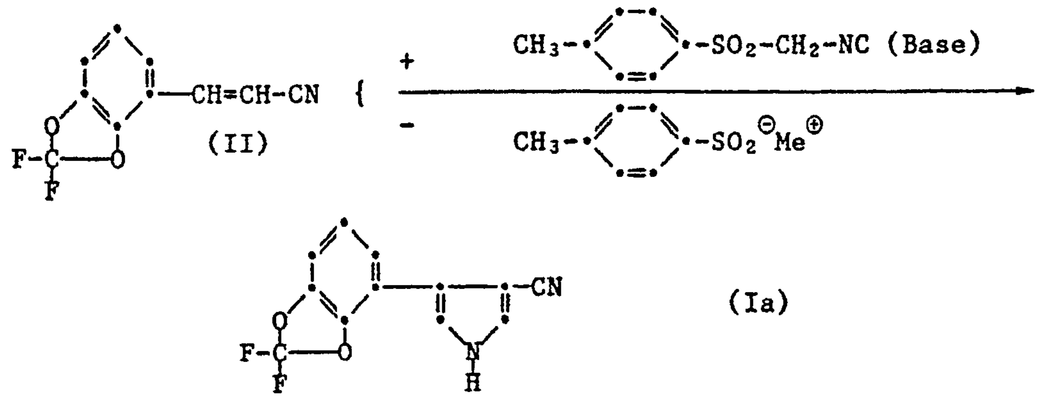

(II)

(Ia)

wobei $Me^{\oplus}$ ein Alkali- oder Erdalkaliion bedeutet, und

b) durch anschliessende Acylierung der Verbindung Ia mit Verbindungen der Formel III in Gegenwart eines säurebindenden Mittels und gegebenenfalls eines Katalysators in einem organischen Lösungsmittel:

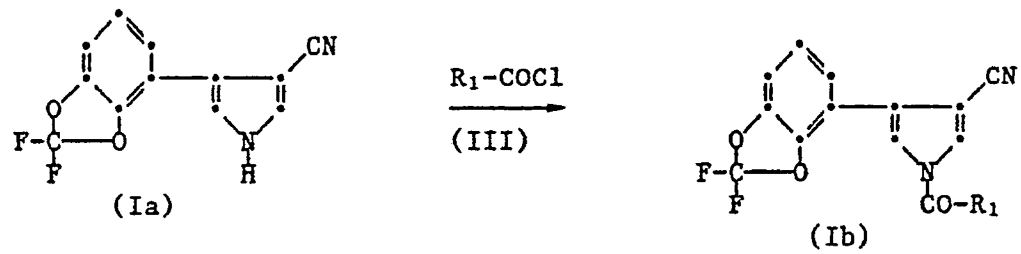


(Ia)

(Ib)

wobei $R^1$ die eingangs unter Formel I angegebenen Bedeutungen hat.

Reaktionsschritt (a):

Die p-Tolylsulfonyl-Gruppe steht hier stellvertretend für eine ganze Reihe von Gruppen, die in der Lage sind, die Methylengruppe im Methylisocyanid-Rest für eine Reaktion im Sinne einer Michael-Addition zu aktivieren. Weitere bevorzugte Beispiele für derartig aktivierende Gruppen sind Benzolsulfonyl-, p-Chlorbenzolsulfonyl, Niederalkylsulfonyl wie Mesyl.

Die Cycloaddition wird vorteilhafterweise in Gegenwart einer nicht nukleophilen Base durchgeführt. Als Basen eignen sich Alkalimetallhydride wie Natriumhydrid oder Alkali- bzw. Erdalkalicarbonate, wie $Na_2CO_3$, $K_2CO_3$ oder Alkalialkoholate, wie $(CH_3)_3CO^{\ominus}K^{\oplus}$ und andere. Die Base wird vorteilhafterweise in mindestens äquimolarer Menge, bezogen auf die Ausgangssstoffe, eingesetzt.

Für die Cycloaddition ist es zweckmässig, die Reaktion in einem reaktionsinternen Lösungsmittel durchzuführen. Dafür kommen beispielsweise folgende, bevorzugt wasserfreie Lösungsmittel in Frage: Aromatische und aliphatische Kohlennwasserstoffe wie Benzol, Toluol, Xylole, Petrolether, Ligroin, Cyclohexan; Ether und etherartige Verbindungen wie Dialkylether (Diethylether, Diisopropylether, t-Butylmethylether usw.), Dimethoxymethan. Dioxan, Tetrahydrofuran, Anisol; Sulfone wie Dimethylsulfoxid; Dimethylformamid und Gemische solcher Lösungsmittel untereinander.

Die Cycloaddition wird im allgemeinen bei Temperaturen von -30° bis +120° C, bevorzugt bei -30° bis +50° C bzw. am Siedepunkt des Lösungsmittels oder Lösungsmittelgemisches, durchgeführt.

Bei der Wahl geeigneter Basen lässt sich die Cycloaddition vorteilhafterweise auch in wässrigem Medium ausführen. Als Basen eignen sich in diesen Fällen wasserlösliche anorganische und organische Basen, insbesondere Alkalihydroxide wie LiOH, NaOH oder KOH und Ammoniumbasen, z.B. Tetraalkylammoniumhydroxide wie $(CH_3)_4NOH$. Die Base wird in mindestens äquimolarer Menge, bezogen auf die Ausgangsstoffe, eingesetzt. Bei der Verwendung wässriger Basen führt man die Reaktion vorteilhafterweise in einem heterogenen Zweiphasensystem durch.

Für die organische, mit Wasser nicht mischbare Phase kommen z.B. folgende Lösungsmittel in Frage: Aliphatische und aromatische Kohlenwasserstoffe, wie Pentan, Hexan, Cyclohexan, Petrolether, Ligroin,

Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw., oder aliphatische Ether, wie Diethylether, Diisopropylether, t-Butylmethylether usw.

Zur Beschleunigung der Reaktionsgeschwindigkeit kann bei dieser Durchführungsart die Gegenwart eines Phasentransfer-Katalysators von Vorteil sein. Beispiele derartiger Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid-, -jodid usw. Als Phasentransfer-Katalysatoren kommen auch Phosphonium-Salze in Betracht.

Die Phasentransfer-katalysierte Cycloaddition kann bei Temperaturen von 0° bis 80° C, vorzugsweise 10° bis 50° C bzw. beim Siedepunkt des Lösungsmittelgemisches, durchgeführt werden.

Die Cycloaddition kann in den beschriebenen Ausführungsarten bei Normaldruck erfolgen; die Reaktionsdauer beträgt im allgemeinen 1 bis 16 Stunden, bei Phasentransferkatalyse 0,5 bis 10 Stunden.

Reaktionsschritt b)

Die Acylierung der Verbindung Ia wird unter üblichen, dem Fachmann bekannten Bedingungen durchgeführt.

Geeignete Lösungs- oder Verdünnungsmittel sind beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Dioxan, Tetrahydrofuran; Nitrile wie Acetonitril, Propionitril; Ketone wie Aceton, Diethylketon, Methylethylketon; und Gemische solcher Lösungsmittel untereinander. Bevorzugt sind Tetrahydrofuran oder Dioxan.

Als säurebindende Mittel eignen sich anorganische Basen, z.B. Oxide, Hydroxide, Carbonate oder Hydrogencarbonate von Alkali- oder Erdalkalimetallen sowie Alkalihydride oder Alkyliacetate, ferner organische Basen, z.B. tertiäre Amine wie Trialkylamine (Trimethylamin, Triethylamin usw.), Pyridin oder Pyridinbasen (4-Dimethylaminopyridin, 4-Pyrrolidylaminopyridin). Bevorzugt sind Trialkylamine wie Trimethylamin oder Triethylamin.

Die Reaktionstemperatur ist je nach den Reaktionsbedingungen variabel. Sie liegt im allgemeinen zwischen -25° C und 100° C, vorzugsweise zwischen -10° und 75° C.

Bei der Herstellung des Zimtsäurenitrils der Formel II als Ausgangsverbindung der Formel Ia geht man von dem 2,3-(Difluormethylendioxy-anilin der Formel IX aus, das in der üblichen, dem Fachmann bekannten Weise zum Diazoniumsalz der Formel X umgesetzt wird:

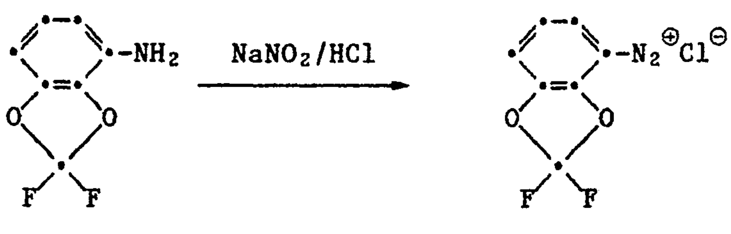


(IX) (X)

Dann lässt man das Diazoniumsalz der Formel X mit dem Acrylnitril der Formel XI in Gegenwart von Cu(I)-Chlorid in einem wässrigen Reaktionsmedium mit einem Dialkylketon als Lösungsvermittler zu dem Anlagerungsprodukt der Formel XII reagieren:

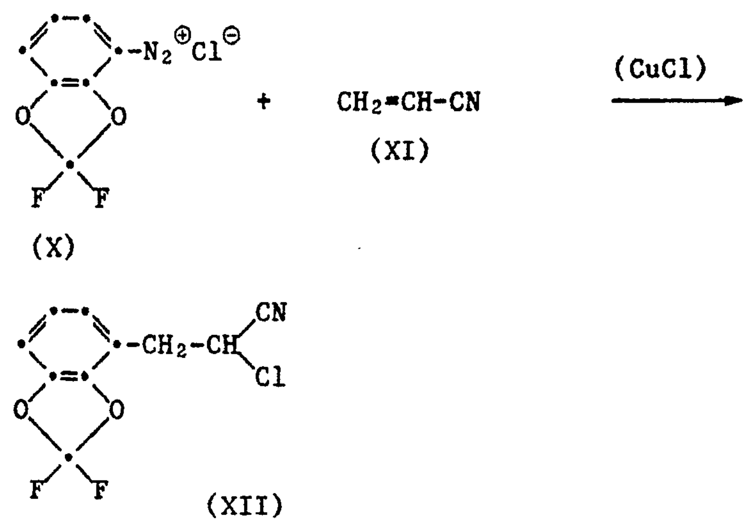


(X) (XI) (XII)

Anschliessend wird durch Umsetzung der Verbindung der Formel XII mit einem säurebindenden Mittel in einem inerten organischen Lösungsmittel HCl abgespalten und dabei das 2,3-(Difluormethylendioxy)-zimtsäurenitril (Formel II) erhalten, wobei das Produkt ein cis/trans-Isomerengemisch darstellt, das chromatographisch in üblicher Weise getrennt werden kann:

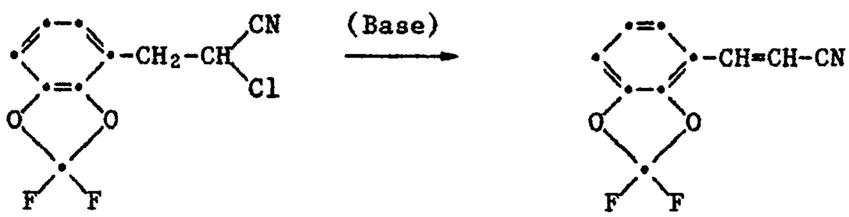


(XII) (II)

Die durchgeführte Umsetzung des Diazoniumsalzes (Formel X) mit dem Acrylnitril (Formel XI) stellt eine Abwandlung der normalen "Sandmeyer"-Methode durch Anwendung der Bedingungen der "Meerwein"-Reaktion von aromatischen Diazonium-Verbindungen auf $\alpha$-,$\beta$-ungesättigte Carbonyl-Verbindungen dar, wodurch der Ersatz der Diazoniumgruppe durch Halogen zu Gunsten der Anlagerungsreaktion zurückgedrängt wird (vgl. E. Müller, Angew. Chemie 61, 178 - 183, 1949).

In der praktischen Durchführung der Umsetzung werden die Reaktanten Diazoniumsalz und Acrylnitril im Verhältnis 1:1 bis 1:8, vorzugsweise 1:2, eingesetzt. Die Reaktionstemperaturen betragen 20° bis 50°C, bevorzugt 25° - 35°C. Die Reaktionsdauer beträgt 0,5 bis 10 Stunden, vorzugsweise 1 bis 3 Stunden. Als Lösungsvermittler im wässrigen Reaktionsmedium wird bevorzugt Ethylmethylketon eingesetzt.

Bei der Abspaltung von HCl aus der Verbindung der Formel XII werden als inerte Lösungsmittel beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander verwendet. Als säurebindende Mittel dienen schwach nucleophile organische Basen, vorzugsweise Trialkylamine. Die Abspaltungsreaktion wird bei Temperaturen, die von Raumtemperatur bis zur Rückflusstemperatur des verwendeten Lösungsmittels reichen, bevorzugt von 30° bis 60°C, durchgeführt. Die Reaktionsdauer beträgt 1 bis 24 Stunden, vorzugsweise 3 bis 12 Stunden.

Die Verbindung der Formel II ist ein wertvolles Zwischenprodukt zur Herstellung von Fungiziden und stellt als neue Verbindung einen Teil der Erfindung dar.

3-Phenyl-4-cyanopyrrolderivate sind bereits z. Teil als Fungizide bekannt. Solche Verbindungen werden

z.B. in Tetrahedron Letters 52, 5337 - 5340 (1972),in den Europäischen Patentanmeldungen EP-A-0 130 149, EP-A-0 133 247 und in der deutschen Offenlegungsschrift DE-OS 29 27 480 beschrieben. Die Wirksamkeit der bekannten Derivate hat sich jedoch nicht immer im gewünschten Masse als voll befriedigend erwiesen.

Es wurde nun überraschend festgestellt, dass die erfindungsgemässen Verbindungen der Formel I ein sehr vorteilhaftes, die praktischen Bedürfnisse gut befriedigendes biozides Wirkungsspektrum gegen schädliche Mikroorganismen, insbesondere gegen phytopathogene Pilze und Bakterien, aufweisen. Sie besitzen sehr vorteilhafte kurative, systemische und insbesondere präventive Eigenschaften und lassen sich zum Schutz von zahlreichen Kulturpflanzen einsetzen. Mit dem Wirkstoff der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Pyricularia und vor allem gegen Botrytis. Botrytis spp. (B. Cinerea, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar. Dabei weist insbesondere die Verbindung Nr. 1.1 aus Tabelle 1 ein breites Wirkungsspektrum auf. Sie entfaltet nicht nur gegen Pyricularia, Botrytis und Rhizoctonia eine hervorragende fungizide Wirkung, sondern eignet sich auch zur erfolgreichen Bekämpfung von Erysiphe- und Venturia-Species. Ueberdies wirken die Verbindungen systemisch. Darüber hinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirksubstanzen zeigen ferner hervorragende Wirkung gegen Boden- und Samen-bürtige Pilze.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Die Wirkstoffe der Formel I können ferner auch als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden, wobei sie sich insbesondere als Getreidebeizmittel in der Bekämpfung von Pilzorganismen, wie beispielsweise Fusarium-, Helminthosporium und Tilletia-Arten, auszeichnen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben): Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja): Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Im Vorratschutz werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Enkapsulierung in z.B. polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Gut; sie hängen jedoch ganz wesentlich

von der Beschaffenheit (Grösse der Oberfläche, Konsistenz, Feuchtigkeitsgehalt) des Gutes und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Agrarprodukte genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him-und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten, Zwiebeln, Tomaten, Kartoffeln, Paprika); Lorbeergewächse (wie Avocadom Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte Zusatzstoffe sind Phospholipide.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g

bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatiscshe Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon,Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht adsorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände, wie z.B. Korkmehl oder Sägemehl, verwendet werden.

Als oberflächenaktive Verbindungen kommen je nach Art des zu formulierenden Wirkstoffes der Formel I nichtionosgene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureessters eines p-Nonylphenol-(4-14-ethylenoxidaddukts in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 g Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherveise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten

niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die agrochemischen Zubereitungen enthalten in der Regel 0,1 bis 99 Gew.%, insbesondere 0,1 bis 95 Gew.% Wirkstoff der Formel I, 99,9 bis 1 Gew.%, insbesondere 99,8 bis 5 Gew.%, eines festen oder flüssigen Zusatzstoffes und 0 bis 25 Gew.% insbesondere 0,1 bis 25 Gew.%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen der Illustration der Erfindung, ohne dieselbe einzuschränken (Prozente und Teile beziehen sich auf Gewicht; Temperaturen sind in Celsiusgraden angegeben).

Herstellungsbeispiele

1.1. Herstellung von 2,3-(Difluormethylendioxy)-zimtsäurenitril

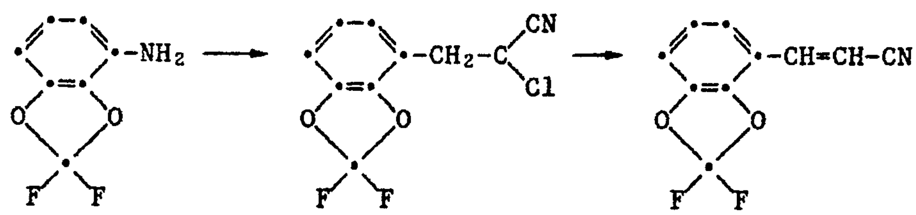

a) Zu einer Lösung von 34.6 g 4-Amino-2,2-difluorbenzodioxol in 71 ml Essigsäure werden 50 ml 32%-ige Salzsäure und 6 ml Wasser gegeben. Zum resultierenden Gemisch wird bei 0° eine Lösung von 15 g Natriumnitrit in 30 ml Wasser zugetropft und anschliessend noch 1 Stunde bei 0° weitergerührt. Die entstandene Suspension wird nun bei 27 - 30° portionenweise zu 27 ml Acrylnitril und 24 ml Aethylmethylketon zufliessen gelassen. Aus einem separaten Tropftrichter wird gleichzeitig eine Lösung von 0.75 g Cu(I)-chlorid in 7.5 ml 32%-iger Salzsäure zugetropft. Nach beendigtem Zutropfen wird noch 30 Min. bei 35° gerührt und dann auf Eis gegossen. Das Gemisch wird zweimal mit Methylenchlorid extrahiert, die organischen Phasen zweimal mit verdünnter, eiskalter Natronlauge extrahiert, über Natriumsulfat getrocknet, filtriert und das Filtrat auf ein Volumen von 700 ml eingeengt.

b) Die obige Methylenchlorid-Lösung wird mit 34.6 ml Triethylamin versetzt und 12 Stunden unter Rückfluss erhitzt. Die dunkle Lösung wird nach dem Abkühlen auf Eiswasser gegossen. Die Phasen werden getrennt und die Wasserphase nochmals mit Methylenchlorid extrahiert. Die organischen Phasen werden zweimal mit eiskalter, verdünnter Salzsäure und anschliessend mit halbgesättigter Natriumchlorid-Lösung gewaschen, über Natriumsulfat getrocknet, filtriert und eingeengt. Durch Chromatographie des rohen cis/trans Gemisches (Petroleumbenzin/Ethylacetat 20:1) kann das zur Hauptsache gebildete trans-Isomere des obigen Zimtsäurenitrils in reiner Form erhalten werden. Gelbliche Kristalle Smp. 53-56°.

NMR (60 MHz, $CFCl_3$) 6.2 ppm (d, J = 17 Hz, 1H);
7.2 ppm (s, 3H),; 7.4 ppm (d, J = 17 Hz, 1H).

1.2. Herstellung von 3-(2,2-Difluorbenzodioxol-4-yl)-4-cyanopyrrol

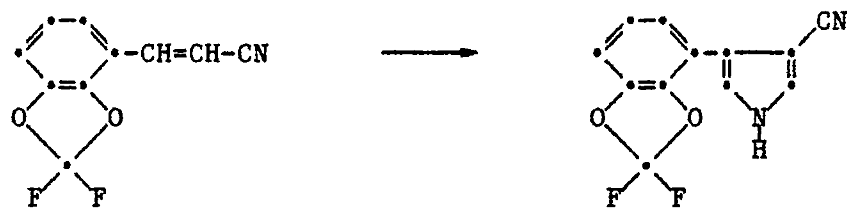

Aus zwei Tropftrichtern werden je eine Lösung von 38.8 g des obigen Zimtsäurenitrils und 43.4 g Tosmic in 250 ml Tetrahydrofuran und von 29.2 g Kalium-tertiär-butylat in 250 ml Tetrahydrofuran, bei -5° bis +5° in 100 ml Tetrahydrofuran getropft. Anschliessend wird 1 Stunde bei 0° und 2 weitere Stunden bei Raumtemperatur gerührt. Das Reaktionsgemisch wird dann auf Eiswasser gegossen und zweimal mit Aethylacetat extrahiert. Die organischen Extrakte werden viermal mit halbgesättigter Natriumchlorid-Lösung gewaschen, über Natriumsulfat getrocknet, mit Kieselgel, etwas Aktivkohle und Tonsil verrührt, filtriert und eingedampft. Der Rückstand wird aus wenig Methylenchlorid bei -30° kristallisiert. Es werden 16.5 g beige Kristalle mit Smp. 197-199° erhalten.

1.3. Herstellung von 1-Acetyl-3-(2,2-Difluor-benzodioxol-4-yl)-4-cyanopyrrol

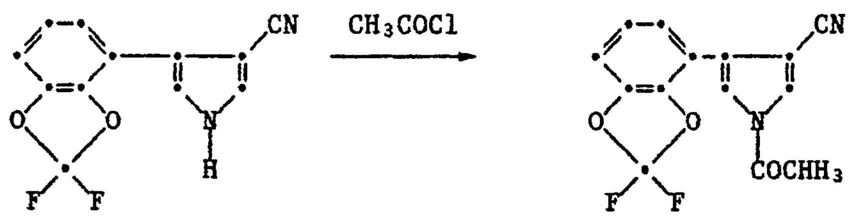


Zu einer Lösung von 2.5 g des obigen Pyrrols in 10 ml Tetrahydrofuran werden 0.2 g 4-Dimethylaminopyridin und 1.6 ml Triethylamin gegeben. Nun wird bei -10° langsam eine Lösung von 0.85 ml Acetylchlorid in 5 ml Tetrahydrofuran zutropfen gelassen. Das Reaktionsgemisch wird 16 Stunden bei auftauendem Eisbad gerührt, filtriert und das Filtrat eingeengt. Der feste Rückstand wird aus Toluol/Petroleumbenzin umkristallisiert. Dabei erhält man kristallines N-(Acetyl)-3-[2,2-Difluoro-benzodioxol-4-yl]-4-cyanopyrrol. Smp. 133-135°.

Auf analoge Weise werden die in der Tabelle angegebenen Verbindungen Nr. 1.3 bis Nr. 1.32 hergestellt.

Tabelle 1

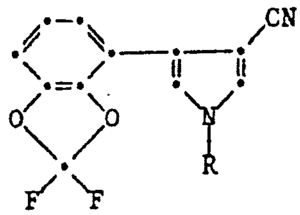

| Nr. | R | chem.-phys. Daten |
|---|---|---|
| 1.1 | H | Fp. 197 - 199° |
| 1.2 | $-COCH_3$ | Fp. 133 - 135° |
| 1.3 | $-COCH_2CH_3$ | Fp. 148 - 150° |
| 1.4 | $-CO-C_3H_7(n)$ | Fp. 133 - 135° |
| 1.5 | $-CO-C_3H_7(c)$ | |
| 1.6 | $-CO-C_4H_9(n)$ | Fp. 122 - 125° |
| 1.7 | $-CO-C_4H_9(s)$ | |
| 1.8 | $-CO-C_4H_9(i)$ | |
| 1.9 | $-CO-C_4H_9(t)$ | Fp. 141 - 143 |
| 1.10 | $-CO-C_5H_4(n)$ | |
| 1.11 | $-CO-C_6H_{13}(n)$ | |
| 1.12 | $-CO-CH_2Cl$ | |
| 1.13 | $-CO-CH_2Br$ | |
| 1.14 | $-CO-CF_3$ | |
| 1.15 | $-CO-CH_2OCH_3$ | Fp. 139 - 141° |
| 1.16 | $-COCH_2CH_2OCH_3$ | |
| 1.17 | $-CO-CH=CH-CH_3$ | Fp. 172 - 174° |
| 1.18 | -CO-cyclopropyl | Fp. 195 - 197° |
| 1.19 | -CO-cyclopentyl | |
| 1.20 | -CO-cyclohexyl | |
| 1.21 | -CO-tetrahydrofur-2-yl | Fp. 116 - 118° |
| 1.22 | $-CO-OCH_3$ | Fp. 143 - 145° |
| 1.23 | $-CO-OCH_2CH_3$ | |
| 1.24 | $-CO-OC_4H_9(n)$ | |
| 1.25 | $-CO-OCH_2CH(CH_3)_2$ | |

Tabelle 1: (Fortsetzung)

| Nr. | R | chem.-phys. Daten |
|---|---|---|
| 1.26 | $-CO-OCH_2CH=CH_2$ | Fp. 126 - 128° |
| 1.27 | $-CO-CH_2-OCH_2CH_2CH_3$ | |

2. Formulierungsbeispiele für flüssige Wirkstoffe der Formel I (% = Gewichtsprozent)

| 2.1. Emulsion-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyäthylenglykoläther (36 Mol Aethylenoxid | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus Tabelle 1 | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

| 2.3. Granulate | a) | b) |
|---|---|---|
| Wirkstoff aus Tabelle 1 | 5% | 10% |
| Kaolin | 94% | |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff aus Tabelle 1 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

| 2.5. Spritzpulver | a) | b) | c) |
|---|---|---|---|
| Wirkstoff aus Tabelle 1 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.6. Emulsions-Konzentrat

| | |
|---|---|
| Wirkstoff aus Tabelle 1 | 10% |
| Octylphenolpolyäthylenglykoläther (4-5 Mol Aethylenoxid) | 3% |
| Ca-Dodecylbenzolsulfonat | 3% |
| Ricinusölpolyglykoläther (35 Mol Aethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff aus Tabelle 1 | 5% | 8% |
| Talkum | 95% | - |
| Kaolin | - | 92% |

Man erhält anwendungsfertige Stäubemittel, indem der Wirkstoff mit dem Träger vermischt auf einer geeigneten Mühle vermahlen wird.

2.8. Extruder Granulat

| | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wurd extrudiert und anschliessend im Luftstrom getrocknet.

2.9. Umhüllungs-Granulat

| | |
|---|---|
| Wirkstoff aus Tabelle 1 | 3% |
| Polyäthylenglykol M G 200 | 3% |
| Kaolin | 94% |

(MG = Molekulargewicht)

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.10. Suspensions-Konzentrat

| | |
|---|---|
| Wirkstoff aus Tabelle 1 | 40% |
| Aethylenglykol | 10% |
| Nonylphenolpolyäthyleglykoläther (15 Mol Aethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele:

Beispiel 3.1.: Wirkung gegen Puccinia graminis auf Weizen

a) Residual-protektive Wirkung
Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Ureidosporensuspension des Pilzes infiziert. Nach einer Inkubation während 48 Stunden bei 95 - 100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.
b) Systemische Wirkung
Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Ureidosporensuspension des Pilzes infiziert. Nach einer Inkubation während 48 Stunden bei 95 - 100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Verbindungen aus der Tabelle 1 zeigten gegen Puccinia-Pilze eine gute Wirkung. Unbehandelte aber infizierte Kontrollpflanzen zeigten einen Puccinia-Befall von 100 %. Unter anderen hemmten die Verbindungen Nr. 1.2, 1.15, 1.22, 1.26, 1.27, den Puccinia-Befall auf 0 bis 5 %.

Beispiel 3.2.: Wirkung gegen Cercospora arachidicola auf Erdnusspflanzen

Residual-protektive Wirkung

10 - 15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus der Tabelle 1 behandelt wurden, einen stark reduzierten Cercospora-Befall. So verhinderten die Verbindungen Nr. 1.1, 1.2, 1.15 in obigen Versuchen das Auftreten von Flecken fast vollständig.

Beispiel 3.3.: Wirkung gegen Erysiphe graminis auf Gerste

Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3 - 4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt. Verbindungen aus der Tabelle 1 waren gut wirksam gegen Erysiphe-Befall von Gerste.

Beispiel 3.4.: Residual-protektive Wirkung gegen Venturia inaequalis auf Apfeltrieben

Apfelstecklinge mit 10 - 20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90 - 100 % relativer Luftfeuchtigkeit inkubiert und während 10 weiteren Tagen in einem Gewächshaus bei 20 - 24°C aufgestellt. Der Schorbefall wird 1 - 5 Tage nach der Infektion beurteilt. Wirkstoffe aus der Tabelle 1 zeigten gute Wirksamkeit gegen Venturia auf Apfeltrieben. Verbindungen Nr. 1.1, 1.2, 1.15, 1.22, 1.26 1.27 hemmten den Krankheitsbefall auf weniger als 10 %. Unbehandelte aber infizierte Triebe zeigten einen 100 %igen Venturia-Befall.

Beispiel 3.5.: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95 - 100 % relativer Luftfeuchtigkeit und 21°C erfolgt die Beurteilung des Pilzbefalls. Die Verbindungen aus der Tabelle 1 hemmten in vielen Fällen die Pilzinfektion sehr stark. Bei einer Konzentration von 0.02 % erwiesen sich z.B. die Verbindungen Nr. 1.1, 1.2, 1.15, 1.22, 1.26, 1.27 als voll wirksam (Krankheitsbefall 0 bis 5 %). Befall unbehandelter aber infizierter Bohnenpflanzen betrug 100 %.

Beispiel 3.6.: Wirkung gegen Botrytis cinerea an Apfelfrüchten

Künstlich verletzte Aepfel werden behandelt, indem eine aus Spritzpulver der Wirksubstanz hergestellte Spritzbrühe auf die Verletzungsstellen aufgetropft wird. Die behandelten Früchte werden anschliessend mit einer Sporensuspension von Botrytis cinerea inokuliert und während einer Woche bei einer hohen Luftfeuchtigkeit und ca. 20°C inkubiert.

Bei der Auswertung werden die angefaulten Verletzungsstellen gezählt und daraus die fungizide Wirkung der Testsubstanz abgeleitet. Verbindungen aus Tabelle 1 waren gut wirksam gegen Botrytis-Befall von Apfelfrüchten. Unter anderen hemmten die Verbindungen Nr. 1.1, 1.2, 1.15, 1.22, 1.26, 1.27 den Pilzbefall im Vergleich zu unbehandelten Kontrollfrüchten (mit 100 % Befall) fast vollständig.

Beispiel 3.7.: Wirkung gegen Alternaria solani auf Tomate

Tomatenpflanzen werden nach dreiwöchiger Anzucht mit einer aus Spritzpulver des Wirkstoffs hergestellten Spritzbrühe (0,06 % Aktivsubstanz) besprüht. Nach 24 Stunden werden die Tomatenpflanzen mit einer Konidiensuspension des Pilzes infiziert. Die Beurteilung der fungiziden Wirkung erfolgt aufgrund des Pilzbefalls nach einer Inkubation der infizierten Pflanzen während 8 Tagen bei hoher Luftfeuchtigkeit und einer Temperatur von 18 - 22°C. Verbindungen aus der Tabelle 1 bewirkten eine stark Reduktion des Alternariabefalls; so hemmten die Substanzen Nr. 1.1, 1.2, 1.15, 1.22, 1.26, 1.27 den Befall vollständig (0 - 5 %).

Beispiel 3.8.: Wirkung gegen Pyricularia oryzae auf Reis

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95 - 100 % relativer Luftfeuchtigkeit und 24°C wird der Pilzbefall beurteilt. Verbindungen aus Tabelle 1 zeigten eine gute Hemmung des

Pyriculariabefalls, so reduzierten z.B. die Verbindungen Nr. 1.1, 1.2, 1.15, 1.22, 1.26, 1.27 den Befall auf weniger als 10 %.

Beispiel 3.9.: Wirkung gegen Fusarium nivale auf Roggen

Auf natürliche Weise mit Fusarium nivale infizierter Roggen der Sorte Tetrahell wird auf einer Mischrolle mit dem zu prüfenden Fungizid gebeizt, wobei eine Konzentration von 60 ppm AS (bezogen auf das Gewicht des Saatgutes) zur Anwendung gelangt. Der infizierte und behandelte Roggen wird im Oktober im Freiland mittels einer Sämaschine auf Parzellen von 3 m Länge und 6 Saatreihen mit dreifacher Wiederholung ausgesät. Bis zur Befallsauswertung wird die Versuchspflanzung unter normalen Feldbedingungen kultiviert (vorzugsweise in einer Region mit geschlossener Schneedecke während der Wintermonate). Zur Ermittlung der Wirkstoffaktivität wird im Frühjahr, unmittelbar nach der Schneeschmelze, der prozentuale Anteil Fusarium-befallener Pflanzen ausgezählt. Verbindungen aus der Tabelle 1 zeigten in diesem Versuch gute Wirksamkeit gegen Fusarium auf Roggen. Unbehandelte jedoch infizierte Kontrollpflanzen wiesen dagegen einen Fusarium-Befall von 100 % auf.

Beispiel 3.10: Wirkung gegen Helminthosporium gramineum an Gerste

Auf natürliche Weise mit Helminthosporium gramineum infizierte Wintergerste der Sorte "CI" wird auf einer Mischrolle mit dem zu prüfenden Fungizid gebeizt, wobei eine Konzentration von 60 ppm AS (bezogen auf das Gewicht des Saatgutes) zur Anwendung gelangt. Die infizierte und behandelte Gerste wird im Oktober im Freiland mittels einer Sämaschine auf Parzellen von 2 m Länge und 3 Saatreihen mit dreifacher Wiederholung ausgesät. Bis zur Befallsauswertung wird die Versuchspflanzung unter normalen Feldbedingungen kultiviert. Zur Ermittlung der Wirkstoffaktivität werden zum Zeitpunkt des Aehrenschiebens der prozentuale Anteil Helminthosporium-befallener Halme ausgezählt. Verbindungen aus Tabelle 1 zeigten in diesem Versuch gute Wirksamkeit gegen Helminthosporium. Unbehandelte jedoch infizierte Kontrollpflanzen wiesen dagegen einen Helminthosporium-Befall von 100 % auf.

Beispiel 3.11: Wirkung gegen Tilletia caries an Weizen

Künstlich mit Brandsporen von Tilletia caries infizierte Winterweizen der Sorte Probus (3 g trockenes Sporenmaterial auf 1 kg Saatgut) wird auf einer Mischrolle mit dem zu prüfenden Fungizid gebeizt, wobei eine Konzentration von 60 ppm AS (bezogen auf das Gewicht des Saatgutes) zur Anwendung gelangt. Der infizierte und behandelte Weizen wird im Oktober im Freiland mittels einer Sämaschine auf Parzellen von 2 m Länge und 3 Saatreihen mit dreifacher Wiederholung ausgesät. Zur Ermittlung der Wirkstoffaktivität werden zum Zeitpunkt der Aehrenreife der prozentuale Anteil Tilletiabefallener Aehren ausgezählt.

Verbindungen aus Tabelle 1 zeigten in diesem Versuch gute Wirksamkeit gegen Tilletia. Unbehandelte jedoch infizierte Kontrollpflanzen wiesen dagegen einen Tilletia-Befall von 100 % auf.

## Patentansprüche

**Patentansprüche für folgende Vertragsstaaten: DE,GB, FR, CH, LI, IT, NL, BE, SE, LU**

**1.** Verbindungen der Formel I

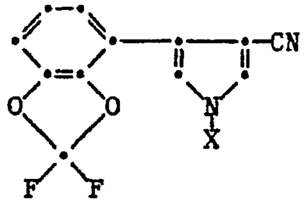

(I)

worin X Wasserstoff oder CO-$R_1$ ist, wobei $R_1$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl; $C_1$-$C_6$-Alkoxy; $C_3$-$C_6$-Alkenyloxy; $C_3$-$C_6$-Cycloalkyl oder Tetrahydrofur-2-yl darstellt.

**2.** Verbindungen der Formel I nach Anspruch 1, worin X Wasserstoff oder $COR_1$ ist, wobei $R_1$ unsubstitu-

iertes $C_1$-$C_4$-Alkyl oder durch Chlor, Brom oder $C_1$ - $C_3$-Alkoxy substituiertes $C_1$-$C_4$-Alkyl; $C_3$-$C_4$-Alkenyl; $C_3$-$C_4$-Alkinyl; $C_1$-$C_4$-Alkoxy; $C_3$-$C_4$-Alkenyloxy; $C_3$-$C_6$-Cycloalkyl oder Tetrahydrofur-2-yl darstellt.

**3.** Eine Verbindung der Formel I nach Anspruch 2, ausgewählt aus der Gruppe:
3-(2,2-Difluor-benzodioxol-4-yl)-4-cyanopyrrol;
1-Acetyl-3-(2,2-difluor-benzodioxol-4-yl)-4-cyanopyrrol;
1-Methoxyacetyl-3-(2,2-difluor-benzodioxol-4-yl)-4-cyanopyrrol;
1-Methoxycarbonyl-3-(2,2-difluor-benzodioxol-4-yl)-4-cyanopyrrol;
1-Allyloxycarbonyl-3-(2,2-difluor-benzodioxol-4-yl)-4-cyanopyrrol;
1-n-Propoxyacetyl-3-(2,2-difluor-benzodioxol-4-yl)-4-cyanopyrrol.

**4.** Verfahren zur Herstellung der Verbindungen der Formel I in Anspruch 1, dadurch gekennzeichnet, dass
a) die Verbindung der Formel II mit p-Toluolsulfonylmethylisocyanid in einem organischen Lösungsmittel in Gegenwart einer Base bei Temperaturen von -30° bis 120° C, vorzugsweise bei -30° bis 50° C, zu einer Verbindung der Formel Ia umgesetzt wird:

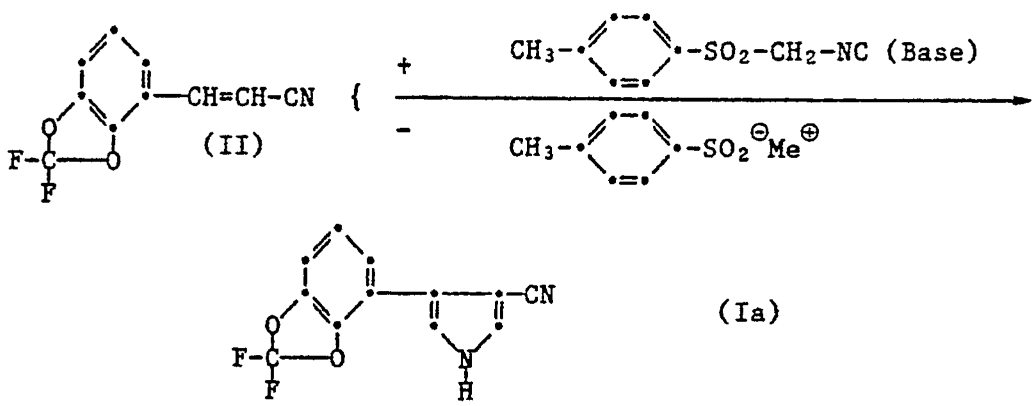


(II) (Ia)

wobei $Me^{\oplus}$ ein Alkali- oder Erdalkaliion bedeutet, und
b) anschliessend die Verbindung der Formel Ia in Gegenwart eines säurebindenden Mittels und gegebenenfalls eines Katalysators in einem organischen Lösungsmittel bei Temperaturen von -25° bis 100° C, vorzugsweise von -10° bis 75° C, acyliert wird:

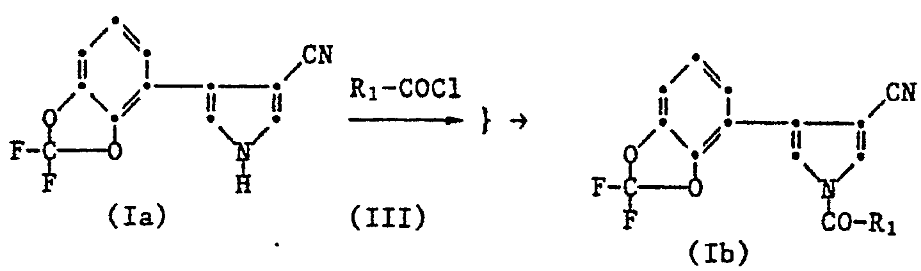


(Ia) (III) (Ib)

wobei $R_1$ die unter Formel I in Anspruch 1 angegebenen Bedeutungen hat.

**5.** Verbindung 2,3-(Difluormethylendioxy)-zimtsäurenitril.

**6.** Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der in Anspruch 1 definierten Verbindungen enthält.

**7.** Mittel gemäss Anspruch 6, dadurch gekennzeichnet, dass es als aktive Komponente mindestens eine der in den Ansprüchen 2 und 3 definierten Verbindungen enthält.

8. Mittel nach einem der Ansprüche 6 oder 7, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

9. Mittel nach Anspruch 8, dadurch gekennzeichnet, dass es 0,1 bis 95 % eines Wirkstoffes der Formel I 99,8 bis 5 % eines festen oder flüssigen Zusatzstoffes und 0,1 bis 25 % eines Tensides enthält.

10. Verfahren zur Herstellung eines in den Ansprüchen 6 bis 9 definierten (agro)chemischen Mittels, dadurch gekennzeichnet, dass man mindestens eine Verbindung der Formel I gemäss einem der Ansprüche 1 bis 3 mit geeigneten festen oder flüssigen Zusatzstoffen und Tensiden innig vermischt.

11. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 3 auf die Pflanze, Pflanzenteile oder deren Standort appliziert.

12. Verfahren zur Beizung von Saatgut und Pflanzenstecklingen zum Schutz gegen den Befall durch Pilzorganismen, dadurch gekennzeichnet, dass man eine Verbindung der Formel I gemäss einem der Ansprüche 1 bis 3 auf das Saatgut oder die Pflanzenstecklinge appliziert.

13. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I gemäss einem der Ansprüche 1 bis 3.

14. Verwendung von Verbindungen der Formel I gemäss Anspruch 1 zur Bekämpfung und/oder zur präventiven Verhütung eines Befalls von Mikroorganismen.

15. Verwendung nach Anspruch 14 von Verbindungen der Formel I gemäss einem der Ansprüche 2 oder 3.

16. Verwendung gemäss einem der Ansprüche 14 oder 15, dadurch gekennzeichnet, dass es sich bei den Mikroorganismen um phytopathogene Pilze handelt.

17. Verwendung gmeäss Anspruch 16 gegen Pilze aus den Klassen Ascomycetes, Basidiomycetes oder Fungi imperfecti.

18. Verwendung gemäss Anspruch 17 gegen Botrytis-Pilze.

**Patentansprüche für folgenden Vertragsstaat: AT**

1. Mikrobizides Mittel enthaltend neben üblichen Träger- und Hilfstoffen eine Verbindung der Formel I

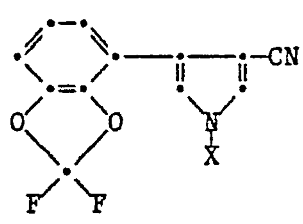

(I)

worin X Wasserstoff oder CO-$R_1$ ist, wobei $R_1$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl; $C_3$-$C_6$-Alkinyl; $C_1$-$C_6$-Alkoxy; $C_3$-$C_6$-Alkenyloxy; $C_3$-$C_6$-Cycloalkyl oder Tetrahydrofur-2-yl darstellt.

2. Mittel nach Anspruch 1 enthaltend eine Verbindung der Formel I, worin X Wasserstoff oder $COR_1$ ist, wobei $R_1$ unsubstituiertes $C_1$-$C_4$-Alkyl oder durch Chlor, Brom oder $C_1$ -$C_3$-Alkoxy substituiertes $C_1$-$C_4$-Alkyl; $C_3$-$C_4$-Alkenyl; $C_3$-$C_4$-Alkinyl; $C_1$-$C_4$-Alkoxy; $C_3$-$C_4$-Alkenyloxy; $C_3$-$C_6$-Cycloalkyl oder Tetrahydrofur-2-yl darstellt.

3. Mittel nach Anspruch 1 enthaltend eine Verbindung der Formel I, ausgewählt aus der Gruppe:
3-(2,2-Difluor-benzodioxol-4-yl)-4-cyanopyrrol;
1-Acetyl-3-(2,2-difluor-benzodioxol-4-yl)-4-cyanopyrrol;
1-Methoxyacetyl-3-(2,2-difluor-benzodioxol-4-yl)-4-cyanopyrrol;
1-Methoxycarbonyl-3-(2,2-difluor-benzodioxol-4-yl)-4-cyanopyrrol;
1-Allyloxycarbonyl-3-(2,2-difluor-benzodioxol-4-yl)-4-cyanopyrrol;
1-n-Propoxyacetyl-3-(2,2-difluor-benzodioxol-4-yl)-4-cyanopyrrol.

4. Verfahren zur Herstellung der Verbindungen der Formel I in Anspruch 1, dadurch gekennzeichnet, dass
a) die Verbindung der Formel II mit p-Toluolsulfonylmethylisocyanid in einem organischen Lösungsmittel in Gegenwart einer Base bei Temperaturen von -30° bis 120° C, vorzugsweise bei -30° bis 50° C, zu einer Verbindung der Formel Ia umgesetzt wird:

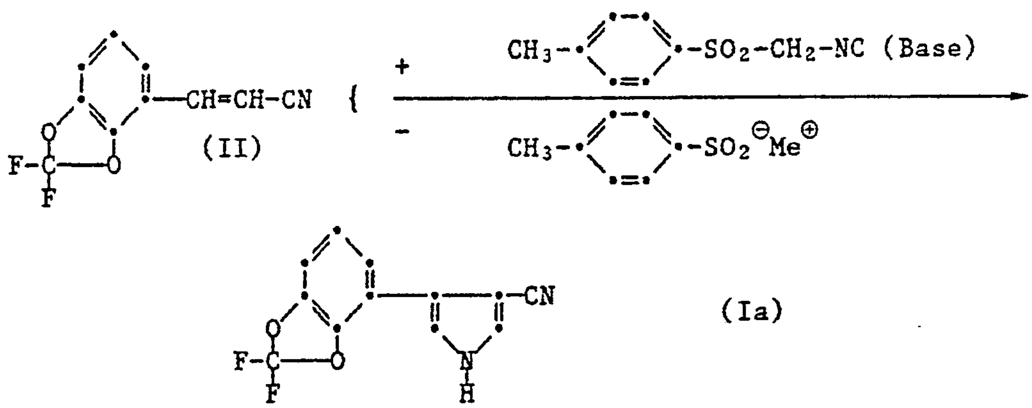
(II)
(Ia)

wobei Me$^{\oplus}$ ein Alkali- oder Erdalkaliion bedeutet, und
b) anschliessend die Verbindung der Formel Ia in Gegenwart eines säurebindenden Mittels und gegebenenfalls eines Katalysators in einem organischen Lösungsmittel bei Temperaturen von -25° bis 100° C, vorzugsweise von -10° bis 75° C, acyliert wird:

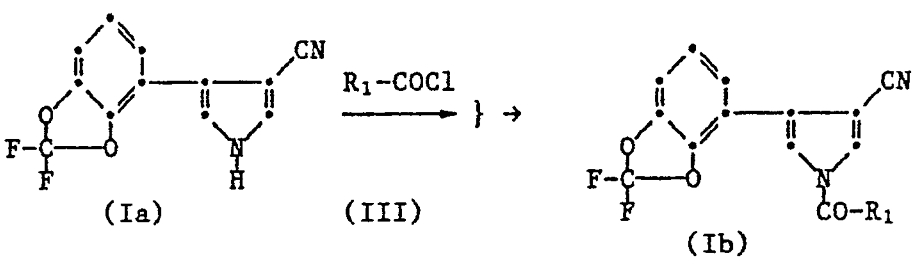

(Ia) (III) (Ib)

wobei $R_1$ die unter Formel I in Anspruch angegebenen Bedeutungen hat.

5. Verwendung der Verbindung 2,3-(Difluormethylendioxy)-zimtsäurenitril zur Herstellung der Verbindungen der Formel I gemäss Anspruch 4.

6. Mittel gemäss einem der Ansprüche 1-3 zur Bekämpfung oder Verhütung eines Befalls von lebenden Pflanzen durch schädliche Mikroorganismen und/oder zur Konservierung von verderblichem Lagergut pflanzlicher oder tierischer Herkunft.

7. Mittel nach einem der Ansprüche 1-3 und 6, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

8. Mittel nach Anspruch 7, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

9. Verfahren zur Bekämpfung oder Verhütung eines Befalls von Kulturpflanzen durch phytopathogene Mikroorganismen, dadurch gekennzeichnet, dass man eine gemäss einem der Ansprüche 1 bis 3 definierte Verbindung der Formel I auf die Pflanze, Pflanzenteile oder deren Standort appliziert.

10. Verfahren zur Beizung von Saatgut und Pflanzenstecklingen zum Schutz gegen den Befall durch Pilzorganismen, dadurch gekennzeichnet, dass man eine gemäss einem der Ansprüche 1 bis 3 definierte Verbindung der Formel I auf das Saatgut oder die Pflanzenstecklinge appliziert.

11. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer gemäss einem der Ansprüche 1 bis 3 definierten Verbindung der Formel I.

12. Verwendung von gemäss Anspruch 4 hergestellten Verbindungen der Formel I zur Bekämpfung und/oder zur präventiven Verhütung eines Befalls von Mikroorganismen.

13. Verwendung nach Anspruch 12 von in einem der Ansprüche 2 oder 3 definierten Verbindungen der Formel I.

14. Verwendung gemäss einem der Ansprüche 11 oder 12 dadurch gekennzeichnet, dass es sich bei den Mikroorganismen um phytopathogene Pilze handelt.

15. Verwendung gemäss Anspruch 14 gegen Pilze aus den Klassen Ascomycetes, Basidiomycetes oder Fungi imperfecti.

16. Verwendung gemäss Anspruch 15 gegen Botrytis-Pilze.

**Claims**

**Claims for the following Contracting States: BE, CH, DE, FR, GB, IT, LI, LU, NL, SE**

1. A compound of the formula I

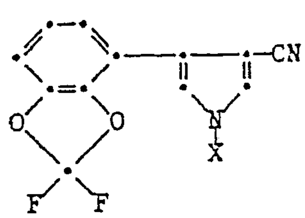

(I)

in which X is hydrogen or CO-$R_1$, where $R_1$ is $C_1$-$C_6$alkyl which is unsubstituted or substituted by halogen or $C_1$-$C_3$alkoxy, or is $C_3$-$C_6$alkenyl, $C_3$-$C_6$alkynyl, $C_1$-$C_6$alkoxy, $C_3$-$C_6$alkenyloxy, $C_3$-$C_6$cycloalkyl or tetrahydrofur-2-yl.

2. A compound of formula I according to claim 1, in which X is hydrogen or $COR_1$, where $R_1$ is $C_1$-$C_4$alkyl which is unsubstituted or substituted by chlorine, bromine or $C_1$-$C_3$alkoxy, or is $C_3$-$C_4$alkenyl, $C_3$-$C_4$alkynyl, $C_1$-$C_4$-alkoxy, $C_3$-$C_4$alkenyloxy, $C_3$-$C_6$cycloalkyl or tetrahydrofur-2-yl.

3. A compound of formula I according to claim 2, selected from the group consisting of
3- (2,2-difluorobenzodioxol-4-yl)-4-cyanopyrrole;
1-acetyl-3-(2,2-difluorobenzodioxol-4-yl)-4-cyanopyrrole;
1-methoxyacetyl-3-(2,2-difluorobenzodioxol-4-yl)-4-cyanopyrrole;
1-methoxycarbonyl-3-(2,2-difluorobenzodioxol-4-yl)-4-cyanopyrrole;
1-allyloxycarbonyl-3-(2,2-difluorobenzodioxol-4-yl)-4-cyanopyrrole;
1-n-propoxyacetyl-3-(2,2-difluorobenzodioxol-4y-l)-4-cyanopyrrole.

4. A process for the preparation of a compound of formula I according to claim 1, which comprises
a) reacting the compound of formula II with p-toluenesulfonylmethyl isocyanide in an organic solvent

in the presence of a base at temperatures from -30° to 120°C, preferably at from -30° to 50°C, to give a compound of formula Ia:

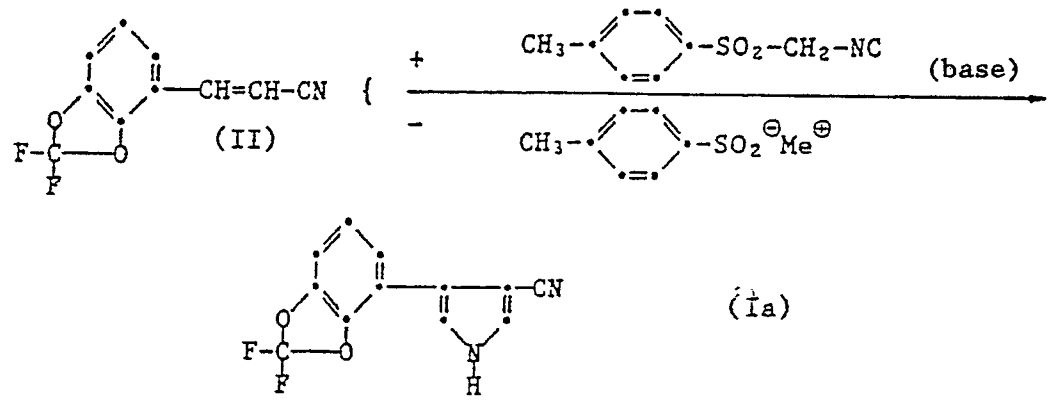

(II) (Ia)

$Me^{\oplus}$ being an alkali metal ion or an alkaline earth metal ion, and
b) subsequently acylating the compound of formula Ia in the presence of an acid-binding agent and optionally of a catalyst in an organic solvent at temperatures from -25° to 100°C, preferably from -10° to 75°C:

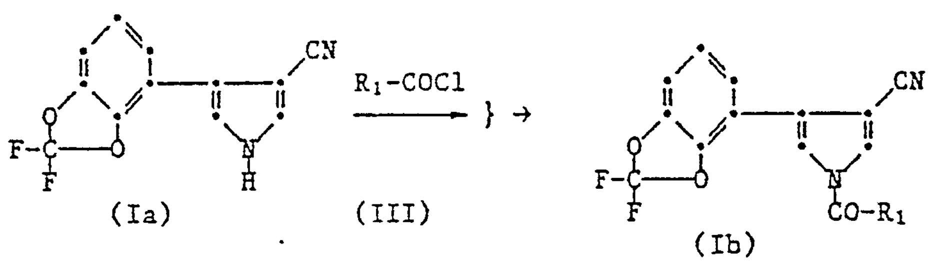

(Ia) (III) (Ib)

$R_1$ being as defined above under formula I in claim 1.

**5.** The compound 2,3-(difluoromethylenedioxy)cinnamonitrile.

**6.** A microbicidal composition for controlling or preventing infestation of living plants and/or for preserving perishable stored goods of vegetable or animal origin, which contains as at least one active component a compound as defined in claim 1.

**7.** A composition according to claim 6, which contains as active component at least one compound as defined in either of claims 2 and 3.

**8.** A composition according to either of claims 6 and 7, which contains 0.1 to 99 % of an active substance of formula I, 99.9 to 1 % of a solid or liquid adjuvant and 0 to 25 % of a surfactant.

**9.** A composition according to claim 8, which contains 0.1 to 95 % of an active substance of formula I, 99.8 to 5 % of a solid or liquid adjuvant and 0.1 to 25 % of a surfactant.

**10.** A process for the preparation of an (agro)chemical composition as defined in any one of claims 6 to 9, which comprises intimately mixing at least one compound of formula I according to any one of claims 1 to 3 with suitable solid or liquid adjuvants and surfactants.

**11.** A method of controlling or preventing infestation of cultivated plants by phytopathogenic microorganisms, which comprises applying to the plants, to parts of plants or to the locus thereof a compound of formula I according to any one of claims 1 to 3.

**12.** A method of dressing seeds and plant cuttings to afford protection against infestation by fungus

organisms, which comprises applying to the seeds or the plant cuttings a compound of formula I according to any one of claims 1 to 3.

**13.** A method of preserving or protecting stock or stored goods of vegetable and/or animal origin from harmful microorganisms by treating the goods with at least a microbicidally effective amount of a compound of formula I according to any one of claims 1 to 3.

**14.** Use of a compound of formula I according to claim 1 for controlling and/or for preventive protection from an infestation by microorganisms.

**15.** Use according to claim 14 of a compound of formula I according to either of claims 2 and 3.

**16.** Use according to either of claims 14 and 15, wherein the microorganisms are phytopathogenic fungi.

**17.** Use according to claim 16 against fungi from the classes Ascomycetes, Basidiomycetes or Fungi imperfecti.

**18.** Use according to claim 17 against Botrytis fungi.

**Claims for the following contracting state: AT**

**1.** A microbicidal composition, containing in addition to conventional carriers and auxiliaries a compound of formula I

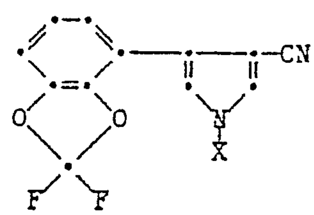

(I)

in which X is hydrogen or CO-$R_1$, where $R_1$ is $C_1$-$C_6$alkyl which is unsubstituted or substituted by halogen or $C_1$-$C_3$alkoxy, or is $C_3$-$C_6$alkenyl, $C_3$-$C_6$alkynyl, $C_1$-$C_6$alkoxy, $C_3$-$C_6$alkenyloxy, $C_3$-$C_6$cycloalkyl or tetrahydrofur-2-yl.

**2.** A composition according to claim 1 containing a compound of formula I, in which X is hydrogen or COR$_1$, where $R_1$ is $C_1$-$C_4$alkyl which is unsubstituted or substituted by chlorine, bromine or $C_1$-$C_3$alkoxy, or is $C_3$-$C_4$alkenyl, $C_3$-$C_4$alkynyl, $C_1$-$C_4$alkoxy, $C_3$-$C_4$alkenyloxy, $C_3$-$C_6$cycloalkyl or tetrahydrofur-2-yl.

**3.** A composition according to claim 1, containing a compound of formula I selected from the group consisting of
3-(2,2-difluorobenzodioxol-4-yl)-4-cyanopyrrole;
1-acetyl-3-(2,2-difluorobenzodioxol-4-yl)-4-cyanopyrrole;
1-methoxyacetyl-3-(2,2-difluorobenzodioxol-4-yl)-4-cyanopyrrole;
1-methoxycarbonyl-3-(2,2-difluorobenzodioxol-4-yl)-4-cyanopyrrole;
1-allyloxycarbonyl-3-(2,2-difluorobenzodioxol-4-yl)-4-cyanopyrrole;
1-n-propoxyacetyl-3-(2,2-difluorobenzodioxol-4-yl)-4-cyanopyrrole.

**4.** A process for the preparation of a compound of formula I according to claim 1, which comprises
a) reacting the compound of formula II with p-toluenesulfonylmethyl isocyanide in an organic solvent in the presence of a base at temperatures from -30° to 120° C, preferably at from -30° to 50° C, to give a compound of formula Ia:

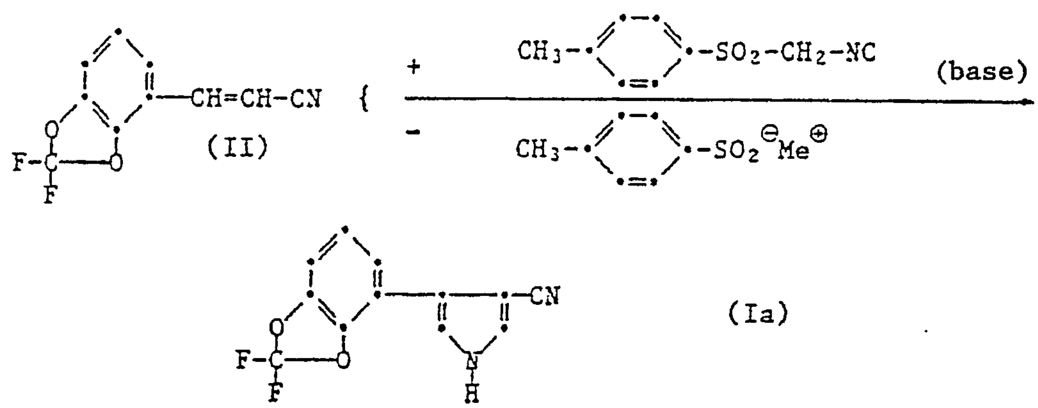

(II) (Ia)

$Me^{\oplus}$ being an alkali metal ion or an alkaline earth metal ion, and

b) subsequently acylating the compound of formula Ia in the presence of an acid-binding agent and optionally of a catalyst in an organic solvent at temperatures from -25° to 100° C, preferably from -10° to 75° C:

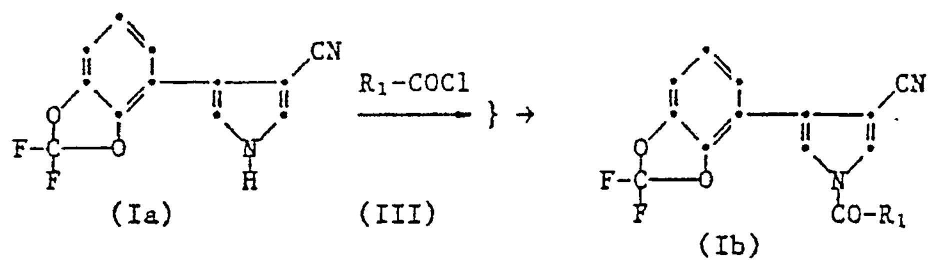

(Ia) (III) (Ib)

$R_1$ being as defined above under formula I in claim 1.

**5.** Use of the compound 2,3-(difluoromethylenedioxy)cinnamonitrile for the preparation of a compound of formula I according to claim 4.

**6.** A composition according to any one of claims 1-3 for controlling or preventing infestation of living plants by harmful microorganisms and/or for preserving perishable stored goods of vegetable or animal origin.

**7.** A composition according to any one of claims 1-3 and 6, which contains 0.1 to 99 % of an active substance of formula I, 99.9 to 1 % of a solid or liquid adjuvant and 0 to 25 % of a surfactant.

**8.** A composition according to claim 7, which contains 0.1 to 95 % of an active substance of formula I, 99.8 to 5 % of a solid or liquid adjuvant and 0.1 to 25 % of a surfactant.

**9.** A method of controlling or preventing infestation of cultivated plants by phytopathogenic microorganisms, which comprises applying to said plants, to parts of plants or to the locus thereof a compound of formula I as defined in any one of claims 1 to 3.

**10.** A method of dressing seeds and plant cuttings to afford protection against infestation by fungus organisms, which comprises applying to the seeds or the plant cuttings a compound of formula I as defined in any one of claims 1 to 3.

**11.** A method of preserving or of protecting stock or stored goods of vegetable and/or animal origin from harmful microorganisms by treating the goods with at least a microbicidally effective amount of a compound of formula I as defined in any one of claims 1 to 3.

**12.** Use of a compound of formula I prepared according to claim 4 for controlling and/or for preventive protection from infestation by microorganisms.

**13.** Use according to claim 12 of a compound of formula I as defined in either of claims 2 and 3.

**14.** Use according to either of claims 11 and 12, wherein the microorganisms are phytopathogenic fungi.

**15.** Use according to claim 14 against fungi from the classes Ascomycetes, Basidiomycetes or Fungi imperfecti.

**16.** Use according to claim 15 against Botrytis fungi.

**Revendications**
**Revendications pour l'Etats contractants suivants: BE, CH, DE, FR, GB, IT, LI, LU, NL, SE**

**1.** Composés de formule I

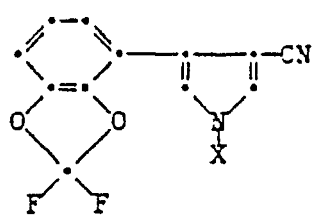

(I)

dans laquelle X représente l'hydrogène ou le groupe CO-$R_1$ dans lequel $R_1$ représente un groupe alkyle en C1-C6 non substitué ou un groupe alkyle en C1-C6 substitué par des halogènes ou des groupes alcoxy en C1-C3; un groupe alcényle en C3-C6, alcynyle en C3-C6; un groupe alcoxy en C1-C6, un groupe alcényloxy en C3-C6; un groupe cycloalkyle en C3-C6 ou tétrahydrofuro-2-yle.

**2.** Composés de formule I de la revendication 1, dans laquelle X représente l'hydrogène ou le groupe CO-$R_1$ dans lequel $R_1$ représente un groupe alkyle en C1-C4 non substitué ou un groupe alkyle en C1-C4 substitué par le chlore, le brome ou des groupes alcoxy en C1-C3; un groupe alcényle en C3-C4; un groupe alcynyle en C3-C4; un groupe alcényloxy en C3-C4; un groupe cycloalkyle en C3-C6 ou tétrahydrofuro-2-yle.

**3.** Un composé de formule I de la revendication 2, choisi dans le groupe suivant :
3-(2,2-difluoro-benzodioxole-4-yl)-4-cyanopyrrole;
1 -acétyl-3-(2,2-difluoro-benzodioxole-4-yl)-4-cyanopyrrole;
1-méthoxyacétyl-3-(2,2-difluoro-benzodioxole-4-yl)-4-cyanopyrrole;
1-méthoxycarbonyl-3-(2,2-difluoro-benzodioxole-4-yl)-4-cyanopyrrole;
1-allyloxycarbonyl-3-(2,2-difluoro-benzodioxole-4-yl)-4-cyanopyrrole;
1-n-propoxyacétyl-3-(2,2-difluoro-benzodioxole-4-yl)-4-cyanopyrrole.

**4.** Procédé de préparation des composés de formule I de la revendication 1, caractérisé en ce que :
a) on convertit le composé de formule II, à l'aide du p-toluène-sulfonylméthylisocyanure dans un solvant organique, en présence d'une base, à des températures de -30 à 120° C, de préférence de -30 à 50° C, en un composé de formule la

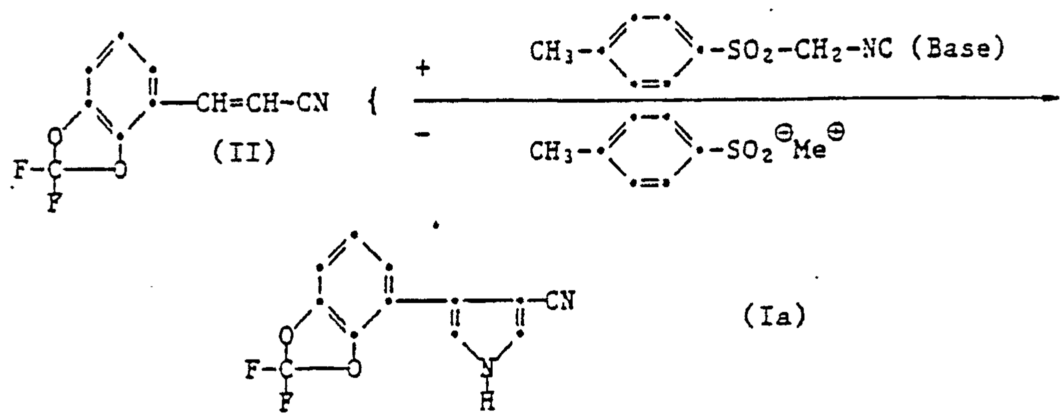


(II) (Ia)

dans laquelle $Me^{\oplus}$ représente un ion alcalin ou alcalinoterreux, et

b) on acyle ensuite le composé de formule Ia en présence d'un accepteur d'acide et le cas échéant d'un catalyseur, dans un solvant organique, à des températures de -25 à 100° C, de préférence de -10 à 75° C :

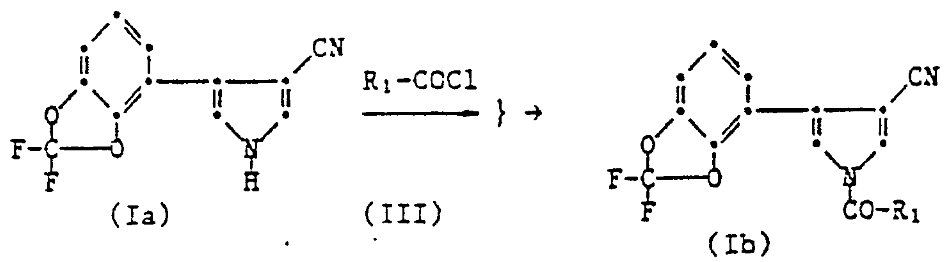


(Ia) (III) (Ib)

$R_1$ ayant dans l'équation ci-dessus les significations indiquées en référence à la formule I dans la revendication 1.

**5.** Le composé : 2,3-(difluorométhylène-dioxy)-cinnamonitrile.

**6.** Produit microbicide prévu pour combattre ou prévenir une infection de végétaux vivants et/ou pour conserver des denrées périssables d'origine végétale ou animale, caractérisé en ce qu'il contient au moins un composant actif consistant en l'un des composés définis dans la revendication 1.

**7.** Produit selon la revendication 6, caractérisé en ce qu'il contient au moins un composant actif consistant en l'un des composés définis dans les revendications 2 et 3.

**8.** Produit selon l'une des revendications 6 ou 7, caractérisé en ce qu il contient de 0,1 à 99 % d'une substance active de formule I, de 99,9 à 1 % d'un additif solide ou liquide et de 0 à 25 % d'un agent tensioactif.

**9.** Produit selon la revendication 8, caractérisé en ce qu'il contient de 0,1 à 95 % d'une substance active de formule I, de 99,8 à 5 % d'un additif solide ou liquide et de 0,1 à 25 % d'un agent tensioactif.

**10.** Procédé de préparation d'un produit (agro)chimique défini dans les revendications 6 à 9, caractérisé en ce que l'on mélange intimement au moins un composé de formule I selon l'une des revendications 1 à 3 avec des additifs solides ou liquides appropriés et des agents tensioactifs.

**11.** Procédé pour combattre ou prévenir une infection de plantes cultivées par des microorganismes phytopathogènes, caractérisé en ce que l'on applique un composé de formule I selon l'une des revendications 1 à 3 sur la plante, des parties de la plante ou son habitat.

12. Procédé pour désinfecter des semences et des pousses de végétaux en vue de les protéger contre l'infection par des mycètes, caractérisé en ce que l'on applique un composé de formule I selon l'une des revendications 1 à 3 sur les semences ou les pousses de végétaux.

13. Procédé pour conserver ou protéger des réserves ou des denrées d'origine végétale et/ou animale contre les microorganismes nuisibles par traitement à l'aide d'un composé de formule I selon l'une des revendications 1 à 3 en quantité microbicide efficace.

14. Utilisation des composés de formule I de la revendication 1 pour combattre et/ou prévenir une infection par des microorganismes.

15. Utilisation selon la revendication 14 de composés de formule I selon une des revendications 2 ou 3.

16. Utilisation selon une des revendications 14 ou 15, caractérisée en ce que les microorganismes sont des mycètes phytopathogènes.

17. Utilisation selon la revendication 16, contre les mycètes des classes des ascomycètes, des basidiomycètes ou des Fungi imperfecti.

18. Utilisation selon la revendication 17, contre les mycètes Botrytis.

**Revendications pour l'Etat contractant suivant: AT**

1. Produit microbicide contenant, avec des véhicules et produits auxiliaires usuels, un composé de formule I

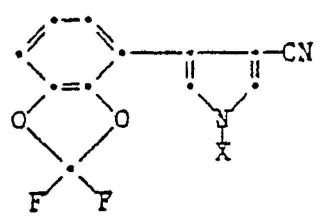

(I)

dans laquelle X représente l'hydrogène ou le groupe CO-$R_1$ dans lequel $R_1$ représente un groupe alkyle en C1-C6 non substitué ou un groupe alkyle en C1-C6 substitué par des halogènes ou des groupes alooxy en C1-C3; un groupe alcényle en C3-C6; un groupe alcynyle en C3-C6; un groupe alcoxy en C1-C6; un groupe alcényloxy en C3-C6; un groupe cycloalkyle en C3-C6 ou un groupe tétrahydrofuro-2-yle.

2. Produit selon la revendication 1, contenant un composé de formule I dans laquelle X représente l'hydrogène ou le groupe CO-$R_1$ dans lequel $R_1$ représente un groupe alkyle en C1-C4 non substitué ou un groupe alkyle en C1-C4 substitué par le chlore, le brome ou des groupes alcoxy en C1-C6; un groupe alcényle en C3-C4; un groupe alcynyle en C3-C4; un groupe alcoxy en C1-C4; un groupe alcényloxy en C3-C4; un groupe cycloalkyle en C3-C6 ou tétrahydrofuro-2-yle.

3. Produit selon la revendication 1, contenant un composé de formule I choisi dans le groupe suivant :
3-(2, 2-difluoro-benzodioxole-4-yl)-4-cyanopyrrole;
1-acétyl-3-(2,2-difluoro-benzodioxole-4-yl)-4-cyanopyrrole;
1-méthoxyacétyl-3-(2,2-difluoro-benzodioxole-4-yl)-4-cyanopyrrole;
1-méthoxycarbonyl-3-(2,2-difluoro-benzodioxole-4-yl)-4-cyanopyrrole;
1-allyloxycarbonyl-3-(2,2-difluoro-benzodioxole-4-yl)-4-cyanopyrrole;
1-n-propoxyacétyl-3-(2,2-difluoro-benzodioxole-4-yl)-4-cyanopyrrole.

4. Procédé de préparation des composés de formule I de la revendication 1, caractérisé en ce que :
a) on convertit le composé de formule II, par réaction avec le p-toluène-sulfonylméthylisocyanure dans un solvant organique, en présence d'une base, à des températures de -30 à 120 ° C, de

préférence de -30 à 50° C, en un composé de formule Ia

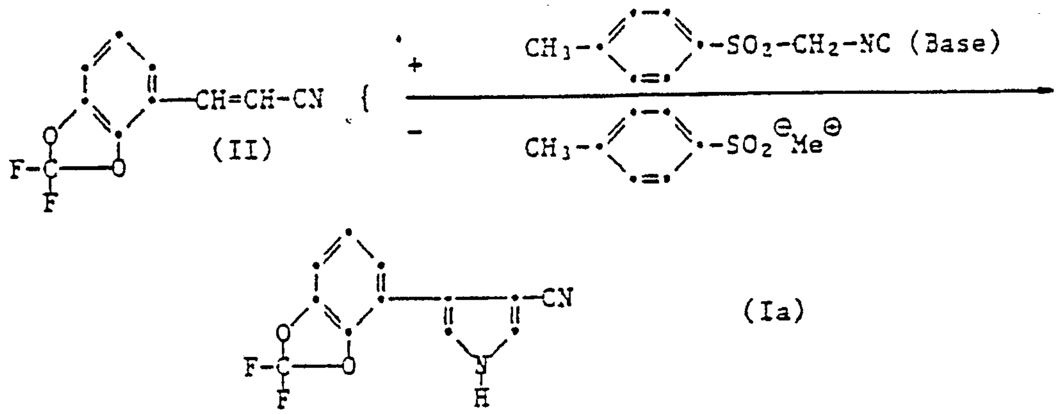


(II)

(Ia)

dans laquelle $Me^{\oplus}$ représente un ion de métal alcalin ou alcalino-terreux, et

b) on acyle ensuite le composé de formule Ia en présence d'un accepteur d'acide et le cas échéant d'un catalyseur, dans un solvant organique, à des températures de -25 à 100° C, de préférence de -10 à 75° C :

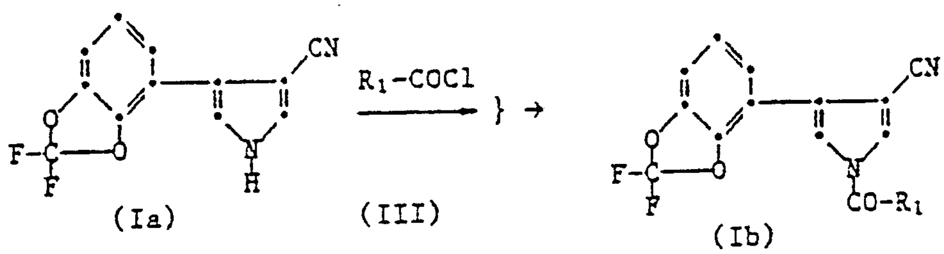


(Ia) (III) (Ib)

$R_1$ ayant dans l'équation ci-dessus les significations indiquées en référence à la formule I dans la revendication 1.

**5.** Utilisation du composé : 2,3-(difluorométhylène-dioxy)-cinnamonitrile pour la préparation des composés de formule I selon revendication 4.

**6.** Produit selon l'une des revendications 1 à 3, pour combattre ou prévenir une infection de végétaux vivants par des microorganismes nuisibles et/ou pour conserver des denrées périssables d'origine végétale ou animale.

**7.** Produit selon l'une des revendications 1 à 3 et 6, caractérisé en ce qu'il contient de 0,1 à 99 % d'une substance active de formule I, de 99,9 à 1 % d'un additif solide ou liquide et de 0 à 25 % d'un agent tensioactif.

**8.** Produit selon la revendication 7, caractérisé en ce qu'il contient de 0,1 à 95 % d'une substance active de formule I, de 99,8 à 5 % d'un additif solide ou liquide et de 0,1 à 25 % d'un agent tensioactif.

**9.** Procédé pour combattre ou prévenir une infection de végétaux cultivés par des microorganismes phytopathogènes, caractérisé en ce que l'on applique un composé de formule I selon l'une des revendications 1 à 3 sur la plante, des parties de la plante ou son habitat.

**10.** Procédé pour désinfecter des semences et les pousses de végétaux qu'on veut protéger contre les infections par les mycètes, caractérisé en ce que l'on applique un composé de formule I défini dans l'une des revendications 1 à 3 sur les semences ou les pousses de végétaux.

**11.** Procédé pour conserver ou protéger des réserves ou des denrées d'origine végétale et/ou animale

contre les microorganismes nuisibles par traitement à l'aide d'au moins un composé défini dans l'une des revendications 1 à 3 en quantité microbicide efficace.

**12.** Utilisation des composés de formule I préparés selon la revendication 4 pour combattre et/ou prévenir une infection par des microorganismes.

**13.** Utilisation selon la revendication 12 de composés de formule I définis dans l'une des revendications 2 ou 3.

**14.** Utilisation selon l'une des revendications 11 ou 12, caractérisée en ce que les microorganismes sont des mycètes phytopathogènes.

**15.** Utilisation selon la revendication 14 contre les mycètes des classes des ascomycètes, des basidiomycètes et des Fungi imperfecti.

**16.** Utilisation selon la revendication 15, contre les mycètes Botrytis.